# EUROPEAN PATENT APPLICATION

(11) **EP 2 343 272 A2**
(43) Date of publication of application: **13.07.2011**
(21) Application number: 09813207.9
(22) Date of filing: 28.08.2009
(51) Int. Cl.: C07C 69/003, C08L 27/06, C08K 5/00

(54) **PLASTICIZER AND POLYVINYL CHLORIDE RESIN COMPOSITION CONTAINING SAME**

(30) Priority: 09.09.2008 KR 20080088707
(71) Applicant: SK Innovation Co. Ltd., Jongno-gu Seoul 110-110 (KR); SK Global Chemical Co., Ltd., Jongro-gu, Seoul 110-110 (KR)
(72) Inventor: KWON, Tae Wook, Daejeon 305-761 (KR); WANG, Hao, Seoul 110-110 (KR); HONG, Seung Gweon, Daejeon 305-728 (KR); KOH, Jae Suk, Daejeon 305-370 (KR)
(74) Representative: Noel, Chantal Odile
(86) International application number: PCT/KR2009/004841
(87) International publication number: WO 2010/030085

(57) **Abstract**

The present invention provides an ester plasticizer, particularly, a plasticizer for a polyvinyl chloride (PVC) resin, and a plastic comprising the plasticizer. More concretely, the present invention provides a plasticizer prepared by the esterification reaction of 1,4 - butanediol and organic acid.

When a polyvinyl chloride resin composition is manufactured using the ester plasticizer of the present invention, there are advantages in that a product having excellent plasticizing efficiency can be obtained, and in that the physical properties such as tensile strength and the like of the product are improved.

## Description

### Technical Field

The present invention relates to an ester compound and a plastic comprising the same, and, particularly, to a plasticizer for a polyvinyl chloride (PVC) resin. More precisely, the present invention relates to an ester plasticizer prepared using the esterification reaction of 1,4-butanediol and a various of carboxylic acid, by which a polyvinyl chloride resin composition having high plasticizing efficiency and improved physical properties such as hardness, tensile strength and the like can be manufactured.

### Background Art

A polyvinyl chloride resin, which is a homopolymer or a copolymer including 50% or more of polyvinyl chloride, is a commonly-used resin which can be formed into various products by extrusion molding, injection molding, calendering or the like. Such a polyvinyl chloride resin is widely used as a raw material for various products, such as pipes, electric wires, electric appliances, toys, films, sheets, synthetic leathers, tarpaulins, tape, packing materials for foods, medical supplies and the like. Various physical properties can be provided to such a polyvinyl chloride resin by suitably adding various additives such as a plasticizer, a stabilizer, filler, pigment and the like.

Among such additives, a plasticizer is an essential additive providing various physical properties and functions, such as workability, flexibility, electrical insulation, adhesivity and the like, to a polyvinyl chloride resin. Low volatility, as a very important factor of a plasticizer, is important both when a plasticizer is mixed in a plastic composition and when a shaped product containing a plasticizer is practically used. Further, plasticizers used in the field of foods, drinks, medical supplies and medicines must be harmless to the human body for health. A typical example of such harmless plasticizers is a phthalate plasticizer. However, it is predicted that the usage of a phthalate plasticizer will be remarkably reduced in the future because of the criticism regarding its toxicity attributable to its reactivation under the laws regulating toxic materials. Therefore, it is required to develop a plasticizer that includes an ester whose basic structure does not contain phthalate and which has a plasticizing efficiency equal to that of a phthalate plasticizer.

### Disclosure

### Technical Problem

In order to solve the above-mentioned problem, the present inventors carefully examined ester compounds that could be used as a plasticizer for a polyvinyl chloride resin. As a result, they found that a specifically-structured novel ester compound can be used as a plasticizer, and, particularly, is excellent as a plasticizer for a polyvinyl chloride resin. Based on these findings, the present invention was completed. Accordingly, an object of the present invention is to provide a novel ester plasticizer prepared using 1,4-butanediol and having physical properties equal to or superior to conventional phthalate plasticizers.

Another object of the present invention is to provide a plasticizer composition comprising the novel ester plasticizer.

Still another object of the present invention is to provide a polyvinyl chloride resin composition comprising the plasticizer composition.

### Technical Solution

In order to accomplish the above objects, a first aspect of the present invention provides an ester plasticizer, represented by Formula 1 below: 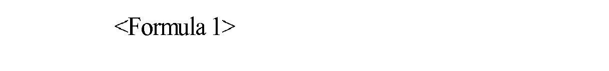 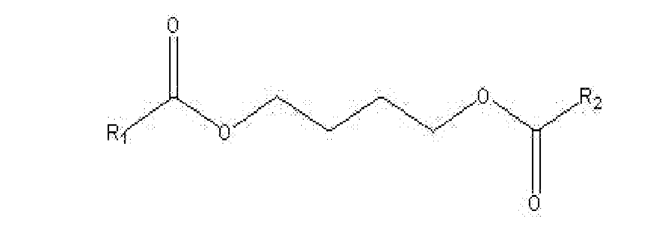 wherein R₁ is a substituted or unsubstituted alkyl group of 4 to 20 carbon atoms, and R₂ is a substituted or unsubstituted aryl group of 6 to 10 carbon atoms.

A second aspect of the present invention provides an ester plasticizer composition, comprising the ester plasticizer by the formula 1 in an amount of 50∼ 100 wt%.

A third aspect of the present invention provides a polyvinyl chloride resin composition, comprising the ester plasticizer composition in an amount of 10 ∼ 100 phr based on a polyvinyl chloride resin.

### Advantageous Effects

When a polyvinyl chloride resin is manufactured using the ester plasticizer of the present invention, there are advantages in that a product having excellent plasticizing efficiency can be obtained, and in that the physical properties such as tensile strength and the like of the product are improved.

### Best Mode

Hereinafter, an ester plasticizer, an ester plasticizer composition comprising the ester plasticizer and a polyvinyl chloride resin composition comprising the ester plasticizer composition according to the present invention will be described in detail.

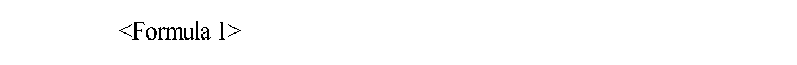

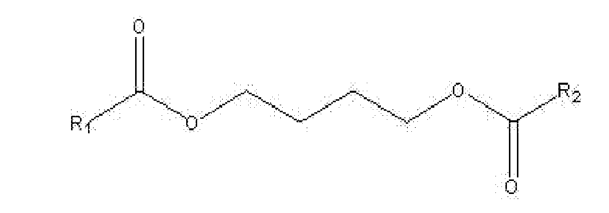

The ester plasticizer of the present invention is a compound represented by Formula 1 above, wherein R₁ is a substituted or unsubstituted alkyl group of 4 to 20 carbon atoms, R₂ is a substituted or unsubstituted aryl group of 6 to 10 carbon atoms, and R₁ and R₂ are different and have an asymmetric structure to each other.

More preferably, R₁ is a substituted or unsubstituted alkyl group of 6 to 10 carbon atoms, and R₂ is a substituted or unsubstituted aryl group of 6 to 8 carbon atoms. R₁ and R₂ each independently include a straight-chain or branched-chain alkyl group of 4 to 20 carbon atoms, an alkenyl group of 4 to 20 carbon atoms, a cycloalkyl group of 4 to 20 carbon atoms or an aryl group of 6 to 10 carbon atoms as a substituent group.

The ester plasticizer is prepared by the esterification reaction of 1,4-butandiol with aromatic carboxylic acid and fatty acid. The molar ratio of aromatic carboxylic acid and fatty acid to 1,4-butanediol is 1: 0.7 - 0.2 : 0.7 - 0.2, preferably 1: 0.4 - 0.25 : 0.6 ∼ 0.3. This molar ratio thereof is determined based on the hydroxy group existing in 1,4-butanediol. It is preferred that an acid catalyst, for example, sodium bisulfate be used in the esterification reaction. Further, p-toluenesulfonic acid, sulfuric acid or the like can be used as the catalyst in the esterification reaction. The catalyst may be used in an amount of 0.5 ∼ 5 wt% based on a reaction mixture.

Meanwhile, examples of the solvents usable in the esterification reaction include hexane, cyclohexane, toluene and xylene. It is preferred that the esterification reaction be conducted at 100 ∼ 160□.

After the esterification reaction, unreacted organic acid and acid catalyst are neutralized by the addition of an alkali reagent such as an aqueous sodium carbonate solution or an aqueous calcium carbonate solution. The coarse ester obtained after phase separation is washed with water, dewatered and then filtered to obtain an object.

The plasticizer composition of the present invention includes the ester plasticizer represented by Formula 1 above in an amount of 50 ∼ 100 wt%. This plasticizer composition may further include a compound represented by Formula 2 below and/or a compound represented by Formula 3 below in an amount of less than 50 wt% based on the total amount of the ester plasticizer composition: 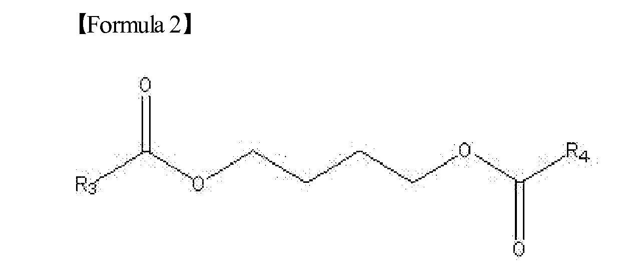 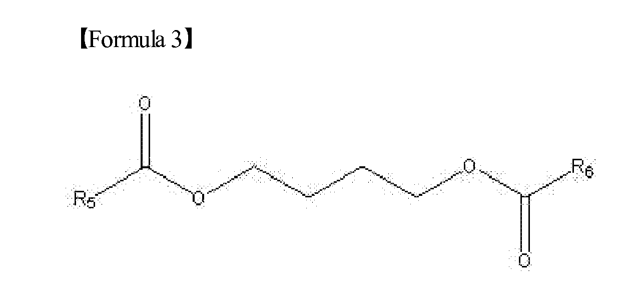 wherein R₃ and R₄ are each independently a substituted or unsubstituted alkyl group of 4 to 20 carbon atoms, R₅ and R₆ are each independently a substituted or unsubstituted aryl group of 6 to 10 carbon atoms, and R₃ to R₆ each independently include a straight-chain or branched-chain alkyl group of 4 to 20 carbon atoms, an alkenyl group of 4 to 20 carbon atoms, a cycloalkyl group of 4 to 20 carbon atoms or an aryl group of 6 to 10 carbon atoms as a substituent group.

The polyvinyl chloride resin composition of the present invention includes the ester plasticizer composition in an amount of 10∼ 100 phr based on a polyvinyl chloride resin.

In the polyvinyl chloride resin composition, the polyvinyl chloride resin is not limited to polyvinyl chloride. Examples of the polyvinyl chloride resin may include: chlorine-containing resins, such as chlorinated polyvinyl chloride, polyvinylidene chloride, chlorinated polyethylene, vinyl chloride acetate copolymer, vinyl chloride ethylene copolymer, vinyl chloride propylene copolymer, vinyl chloride styrene copolymer, vinyl chloride isobutylene copolymer, vinyl chloride vinylidene copolymer, vinyl chloride and various kinds of ether copolymers, and blends thereof; and blends, block copolymers and graft copolymers of the chlorine-containing resins and resins containing no chlorine for example acylonitrile-styrene copolymer, acrylonitrile-styrene-butadiene terpolymer, ethylene-vinyl acetate copolymer, polyesters, etc.

In the polyvinyl chloride resin composition of the present invention, the amount of the ester plasticizer composition may be suitably adjusted according to the use of polyvinyl chloride resin composition. When the amount of the ester plasticizer composition is less than 10 phr, flexibility or workability, which can be exhibited by a plasticizer, cannot be realized. Further, when the amount thereof is greater than 100 phr, it is difficult to ensure necessary mechanical properties, and it is probable that the polyvinyl chloride resin composition will elute.

Meanwhile, the polyvinyl chloride resin composition may further include additives, excluding filler and pigment, in an amount of 0 - 30 phr based on the polyvinyl chloride resin.

Here, examples of the additives, which are general additives that can be selectively included in the polyvinyl chloride resin composition, may include an insulation improver, various kinds of metal salts, polyols, epoxy compounds, a phenolic or sulfuric antioxidant, an ultraviolet absorber, a hindered amine-based photostabilizer, an inorganic stabilizer, an antifogging agent, an anti-misting agent, an auxiliary stabilizer, organic tin compounds, and the like.

Unlike the general additives, filler and pigment can be included up to about 200 phr based on the polyvinyl chloride resin. When the amount of the filler and pigment is greater than 200 phr, the density, hardness or flexibility of the polyvinyl chloride resin composition is negatively influenced. Examples of the filler may include calcium carbonate, silica, clay, glass beads, mica, sericite, glass flakes, asbestos, wollastonite, potassium titanate, polarization-maintaining fiber (PMF), gypsum fiber, xonotlite, metal-oxide semiconductor (MOS), phosphate fiber, glass fiber, carbon fiber, aramid fiber, and the like.

The polyvinyl chloride resin composition including the ester plasticizer of the present invention can be used in building materials, such as wall-finishing materials, floor materials, window sashes, wallpaper, and the like; wire covering materials; interior and exterior materials for automobiles; agricultural materials, such as materials for vinyl houses, tunnels and the like; food wrappers; film-forming agents, such as sealant, plastisol, paint, ink and the like; and miscellaneous goods, such as synthetic leather, coated fabrics, hoses, pipes, sheets, toys for infants, gloves and the like. However, the present invention is not limited thereto.

Methods of preparing the polyvinyl chloride resin composition using the ester plasticizer composition are not particularly limited, and are well known in the related field.

### Mode for Invention

Hereinafter, the present invention will be described in more detail with reference to the following Examples. However, these Examples are set forth to illustrate the present invention, and the scope of the present invention is not limited thereto. In these Examples, the physical properties of samples were evaluated by the following method.

### Hardness

Based on the ASTM D2240, the needle of a hardness tester (A type) was completely pressed onto one point of a sample for 5 seconds, and then the hardness of the sample was measured. Hardness tests were conducted at three points of each sample, and then the average value thereof was obtained. Hardness is used as an index for representing plasticizing efficiency.

### Tensile strength, elongation, elastic modulus at 100% elongation

The tensile strength and elastic modulus of a sample were measured using UTM based on the ASTM D412 method. The tensile strength and elastic modulus thereof were measured at the cut point of a dumbbell-shaped sample after it was pulled at a crosshead speed of 200 mm/min. The elastic modulus at 100% elongation corresponds to the tensile strength at 100% elongation, and is closely related to plasticizing efficiency.

### Maximum torque

The maximum torque occurring at the time of mixing a polyvinyl chloride with a plasticizer was measured using a Brabender tester.

### [Example 1]

### Preparation of an ester plasticizer using 1,4-butanediol, benzoic acid and decanoic acid

First, 1.0 mol of 1,4-butanediol, 1.0 mol of benzoic acid, 1.0 mol of decanoic acid, 200 g of toluene as a solvent, and 3.0 g of sodium bisulfate as a catalyst were put into a 2L round flask provided with a stirrer and a condenser, and were then heated to 130 °C to conduct a reaction for 12 hours.

After the reaction, unreacted organic acid and acid catalyst were neutralized by 10 wt% of an aqueous sodium carbonate solution, water-washed, dewatered and then filtered by an adsorbent to obtain an ester plasticizer including benzoic acid 4-decanoyloxy-butyl ester (about 50 wt%) as a main component.

### Preparation of a polyvinyl chloride resin composition

Test samples were fabricated in order to evaluate the performance of the obtained ester plasticizer. That is, 50 phr of the obtained ester plasticizer including benzoic acid 4 - decanoyloxy-butyl ester as a main component and 1 phr of a stabilizer (LFX-1100) were mixed with a polyvinyl chloride resin (LS-100, manufactured by LG Chemicals Co., Ltd.)of part by weight, and then the mixture was preheated to 185 °C for 1 minute, pressurized for 1.5 minutes and cooled for 2 minutes to obtain a sheet having a thickness of 2 mm The sheet was formed into various dumbbell-shaped test samples.

The above-mentioned tests were conducted using these test samples, and the results thereof are given in Table 1 below.

### [Example 2]

An ester plasticizer and a polyvinyl chloride resin composition were prepared in the same manner as Example 1, except that octanoic acid was used instead of decanoic acid. The test results thereof are given in Table 1 below.

### [Example 3]

An ester plasticizer and a polyvinyl chloride resin composition were prepared in the same manner as Example 1, except that dodecanoic acid was used instead of decanoic acid. The test results thereof are given in Table 1 below.

### [Example 4]

An ester plasticizer and a polyvinyl chloride resin composition were prepared in the same manner as Example 1, except that 2-ethylhexanoic acid was used instead of decanoic acid. The test results thereof are given in Table 1 below.

### [Comparative Example 1]

A test sample was fabricated in the same manner as Example 1 using di-2-ethylhexyl phthalate as a plasticizer. The same test that had been conducted in Example 1 was conducted using this test sample, and the results thereof are given in Table 1 below.

### [Comparative Example 2]

A test sample was fabricated in the same manner as Example 1 using diisononyl phthalate as a plasticizer instead of di-2-ethylhexyl phthalate. The same test that had been conducted in Example 1 was conducted using this test sample, and the results thereof are given in Table 1 below.

### [Comparative Example 3]

A test sample was fabricated in the same manner as Example 1 using trioctyl trimellitate as a plasticizer. The same test that had been conducted in Example 1 was conducted using this test sample, and the results thereof are given in Table 1 below.

**[Table 1]**

| Measured items | Ex. 1 | Ex. 2 | Ex. 3 | Ex. 4 | Co. Ex. 1 | Co. Ex. 2 | Co. Ex. 3 |
|---|---|---|---|---|---|---|---|
| Hardness (Shore A) | 77 | 73 | 79 | 74 | 79 | 84 | 88 |
| Tensile strength (Kgf/cm²) | 210 | 190 | 204 | 195 | 198 | 207 | 214 |
| Elongation (%) | 434 | 390 | 390 | 380 | 350 | 356 | 350 |
| Modulus (Kgf/cm²) | 70 | 63 | 77 | 67 | 87 | 101 | 119 |
| Maximum torque (Nm) | 4.3 | 4.4 | 3.8 | 4.3 | 4.4 | 4.5 | 4.4 |

From the results of Table 1 above, it can be presumed that the plasticizing efficiency of the plasticizers of Examples 1, 2 and 4 is higher than that of the plasticizers of Comparative Examples 1, 2 and 3, and that the physical properties, such as tensile strength, elongation and the like, of the plasticizers of Examples 1, 2 and 4 are equal to or greater than those of the plasticizers of Comparative Examples 1,2 and 3. Further, it can be presumed that the plasticizer of Example 3 is similar to the plasticizer of Comparative Example 1 in terms of hardness deeply related to plasticizing efficiency. Therefore, it is expected that, since the novel plasticizer of the present invention has high plasticizing efficiency, it can be formed in various shapes, and thus it can be variously utilized.

As described above, although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An ester plasticizer, represented by Formula 1 below: 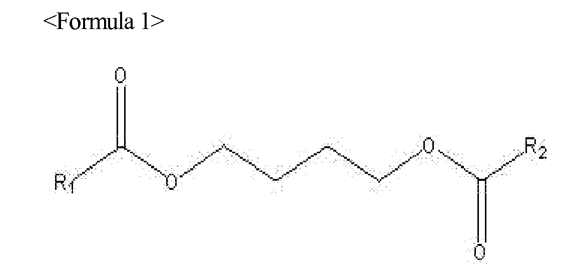 wherein R₁ is a substituted or unsubstituted alkyl group of 4 to 20 carbon atoms, and R₂ is a substituted or unsubstituted aryl group of 6 to 10 carbon atoms.

2. The ester plasticizer according to claim 1, wherein R₁ is a substituted or unsubstituted alkyl group of 6 to 10 carbon atoms, and R₂ is a substituted or unsubstituted aryl group of 6 to 8 carbon atoms.

3. The ester plasticizer according to claim 1, wherein R₁ and R₂ each independently include a straight-chain or branched-chain alkyl group of 4 to 20 carbon atoms, an alkenyl group of 4 to 20 carbon atoms, a cycloalkyl group of 4 to 20 carbon atoms or an aryl group of 6 to 10 carbon atoms as a substituent group.

4. An ester plasticizer composition, comprising the ester plasticizer of claim 1 in an amount of 50 ∼ 100 wt%.

5. The ester plasticizer composition according to claim 4, further comprising at least one of compounds represented by Formulae 2 and 3 below in an amount of less than 50 wt% based on the total amount of the ester plasticizer composition: 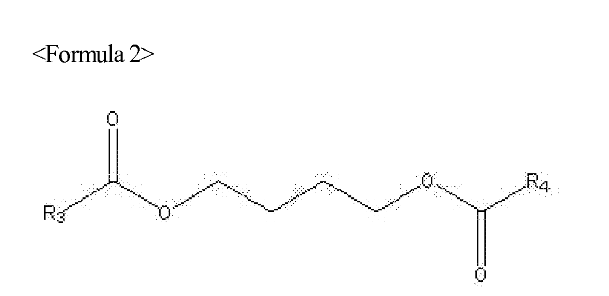 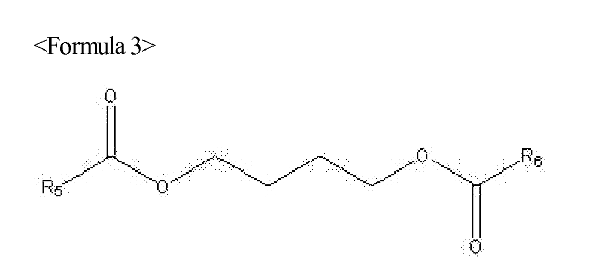 wherein R₃ and R₄ are each independently a substituted or unsubstituted alkyl group of 4 to 20 carbon atoms, and R₅ and R₆ are each independently a substituted or unsubstituted aryl group of 6 to 10 carbon atoms.

6. The ester plasticizer composition according to claim 5, wherein R₃ to R₆ each independently include a straight-chain or branched-chain alkyl group of 4 to 20 carbon atoms, an alkenyl group of 4 to 20 carbon atoms, a cycloalkyl group of 4 to 20 carbon atoms or an aryl group of 6 to 10 carbon atoms as a substituent group.

7. A polyvinyl chloride resin composition, comprising the ester plasticizer composition of any one of claims 4 to 6 in an amount of 10 ∼ 100 phr based on a polyvinyl chloride resin.

8. The polyvinyl chloride resin composition according to claim 7, further comprising additives, excluding filler and pigment, up to 30 phr based on a polyvinyl chloride resin.
